# EUROPEAN PATENT SPECIFICATION

(11) **EP 4 185 993 B1**
(45) Date of publication and mention of the grant of the patent: **14.10.2026**
(21) Application number: 21846558.1
(22) Date of filing: 19.07.2021
(51) Int. Cl.: G06T 19/20

(54) **SYSTEMS AND METHODS FOR MODELING DENTAL STRUCTURES**
SYSTEME UND VERFAHREN ZUR MODELLIERUNG VON ZAHNSTRUKTUREN
SYSTÈMES ET PROCÉDÉS DE MODÉLISATION DE STRUCTURES DENTAIRES

(30) Priority: 21.07.2020 US 202063054712 P
(43) Date of publication of application: 31.05.2023
(73) Proprietor: Get-Grin Inc., Airmont, NY 10952 (US)
(72) Inventor: LIPNIK, Alon Luis, New York 10952 (US); KORETZ, Amitai, New York 10952 (US); SCHULHOF, Adam Benjamin, New York 10952 (US)
(74) Representative: J A Kemp LLP
(86) International application number: PCT/US2021/042247
(87) International publication number: WO 2022/020267

(56) References cited:
- US-A1- 2009 076 321
- US-A1- 2014 272 764
- US-A1- 2016 373 155
- US-A1- 2019 313 963
- US-A1- 2020 000 551
- US-B1- 6 402 707
- TAMAYO-QUINTERO J D ET AL: "Image Segmentation Techniques Applied to Point Clouds of Dental Models with an Improvement in Semi-Automatic Teeth Segmentation", 1 January 2014 (2014-01-01), pages 1, XP093177334, Retrieved from the Internet <URL:https://worldcomp-proceedings.com/proc/p2014/IPC2502.pdf>

## Description

### CROSS REFERENCE

This application claims priority to U.S. Provisional Application No. 63/054,712 filed on July 21, 2020.

### BACKGROUND

Dental professionals and orthodontists may treat and monitor a patient's dental condition based on in-person visits. Treatment and monitoring of a patient's dental condition may require a patient to schedule multiple in-person visits to a dentist or orthodontist. The quality of treatment and the accuracy of monitoring may vary depending on how often and how consistently a patient sees a dentist or orthodontist. In some cases, suboptimal treatment outcomes may result if a patient is unable or unwilling to schedule regular visits to a dentist or orthodontist. The following document describes background art: US 2020/000551 A1. A1, in which a 3d parametric model of a dental structure is obtained by varying the parameters and comparing the resulting 2d silhouette to the silhouette of the 2D image of the teeth.

### SUMMARY

The invention is defined in the appended independent claims. Any aspects, examples or embodiments falling outside the scope of the claims do not form part of the invention, and are provided for illustrative purposes. Recognized herein is a need for remote dental monitoring solutions to allow dental patients to receive high quality dental care, without requiring a dental professional to be physically present with the patient, and without requiring a clinical intraoral scanner. In particular, there is a need for methods and systems that can accurately build a three-dimensional (3D) model of a dental structure of the patient using an existing user device (e.g., mobile device, smartphone), and can be used in a variety of places without time consuming or expensive setup processes. The 3D model may provide patients and dentists with a precise, current and manipulatable 3D image of the patient's complete dental structure for determining a dental condition of the subject, diagnostic and treatment planning purposes or various other purposes.

The present disclosure provides methods and systems that are capable of generating (or configured to generate) a high-quality three-dimensional (3D) model of a dental structure of a dental patient using images (e.g., camera image, camera video, *etc.)* collected using a mobile device. The high-quality 3D model may be a 3D surface model (mesh) with fine details of the surface of the dental structure. The high-quality 3D model reconstructed from the camera images as described herein can have substantially the same or similar quality and surface details as those of a 3D model (e.g., optical impressions) produced using an existing high-resolution clinical intraoral scanner. It is noted that high-resolution clinical intraoral scans can be time-consuming and uncomfortable to the patient. Methods and systems of the present disclosure beneficially provide a convenient and efficient solution for monitoring and evaluating the positions of a patient's teeth during the course of orthodontic treatment using a user mobile device, in the comfort of the patient's home or another convenient location, without requiring the patient to travel to a dental clinic or undergo a time-consuming and uncomfortable full clinical intraoral dental scan.

In an aspect, the present disclosure provides methods for generating a high-quality 3D surface model. The method may comprise: capturing image data about the dental structure of the subject using a camera of a mobile device; constructing a first 3D model of the dental structure from the image data; registering the first 3D model with an initial 3D surface model to determine a transformation for at least one element of the dental structure; and updating the initial 3D surface model by (i) applying the transformation to update a position of the at least one element and/or (ii) deforming a surface of a local area of the at least one element using a deformation algorithm.

In another aspect, the present disclosure provides a "reconstruction free" method based on differentiable-rendering. Such a method provides an alternative to the construction of the first 3D model and subsequent registration to the initial 3D surface model. The "reconstruction free" method can be used to estimate a movement of one or more dental features over a target time period. In some cases, such target time period may be predetermined. In other cases, such target time period may be adjustable based on an input from a patient or a dental practitioner (e.g., an input corresponding to a desired target time period), the patient's current or historical progress with respect to a dental treatment plan, or a current stage of the dental treatment plan. In some cases, the movement of the one or more dental features may correspond to a relative tooth motion. The relative motion may be determined based on a comparison between a 3D scan (e.g., a 3D intraoral scan captured using a clinical dental scanner) and a 2D video scan (e.g., a 2D intraoral video scan captured at a later point in time using a mobile device).

In another aspect, the present disclosure provides a method for generating a three-dimensional (3D) model of a dental structure of a subject, comprising: (a) capturing image data associated with the dental structure of the subject using a camera of a mobile device; (b) processing the image data using an image processing algorithm, wherein the image processing algorithm is configured to implement differentiable rendering; and (c) using the processed image data to generate a 3D surface model corresponding to one or more dental features represented in the image data. In some embodiments, processing the image data comprises comparing the image data to one or more two-dimensional (2D) renderings of a three-dimensional (3D) mesh associated with the dental structure of the subject. In some embodiments, the method may further comprise applying one or more rigid transformations to align or match at least a portion of the image data to the one or more 2D renderings of the 3D mesh associated with the dental structure of the subject. In some embodiments, the one or more rigid transformations comprise a six degree of freedom rigid transformation. In some embodiments, the method may further comprise evaluating or quantifying a level of matching using an intersection-over-union metric. In some embodiments, the method may further comprise determining a movement of one or more dental features based on the comparison between the image data and the one or more 2D renderings of the 3D mesh associated with the dental structure of the subject. In some embodiments, the method may further comprise, in step (a), providing visual, audio, or haptic guidance to aid in the capture of the image data. In some embodiments, the guidance corresponds to a position, an orientation, or a movement of the mobile device relative to the dental structure of the subject.

Another aspect of the present disclosure provides a non-transitory computer readable medium comprising machine executable code that, upon execution by one or more computer processors, implements any of the methods disclosed herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1** shows an example of a 3D model reconstruction algorithm, in accordance with some embodiments of the present disclosure;
**FIG. 2** shows an example of a user device for capturing intraoral image data;
**FIG. 3** shows an exemplary algorithm for building a reduced 3D model from multiple intraoral images or videos;
**FIG. 4** shows an example of a reduced 3D model (e.g., dense 3D point cloud) reconstructed from the camera image;
**FIG. 5** shows an example of a method for determining the transformation parameters;
**FIG. 6** shows an example of a 3D surface model that is obtained from an initial clinical intraoral scan and an example of registration result;
**FIG. 7** shows an example of a registration result;
**FIG. 8** illustrates an example of a surface deformation algorithm, in accordance with some embodiments of the present disclosure;
**FIG. 9** shows an example of updating the initial mesh model by updating the position of a shifted tooth to the new position;
**FIG. 10** shows an example of updating the initial mesh model to generate a new 3D surface model; and
**FIG. 11** illustrates an exemplary environment in which a remote dental monitoring and imaging system described herein may be implemented.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The term "real-time," as used herein, generally refers to a simultaneous or substantially simultaneous occurrence of a first event or action with respect to an occurrence of a second event or action. A real-time action or event may be performed within a response time of less than one or more of the following: ten seconds, five seconds, one second, a tenth of a second, a hundredth of a second, a millisecond, or less relative to at least another event or action. A real-time action may be performed by one or more computer processors.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

The terms "a," "an," and "the," as used herein, generally refer to singular and plural references unless the context clearly dictates otherwise.

Reference throughout this specification to "some embodiments," or "an embodiment," means that a particular feature, structure, or characteristic described in connection with the embodiment is included in at least one embodiment. Thus, the appearances of the phrase "in some embodiment," or "in an embodiment," in various places throughout this specification are not necessarily all referring to the same embodiment. Furthermore, the particular features, structures, or characteristics may be combined in any suitable manner in one or more embodiments.

As utilized herein, terms "component," "system," "interface," "unit" and the like are intended to refer to a computer-related entity, hardware, software (e.g., in execution), and/or firmware. For example, a component can be a processor, a process running on a processor, an object, an executable, a program, a storage device, and/or a computer. By way of illustration, an application running on a server and the server can be a component. One or more components can reside within a process, and a component can be localized on one computer and/or distributed between two or more computers.

Further, these components can execute from various computer readable media having various data structures stored thereon. The components can communicate via local and/or remote processes such as in accordance with a signal having one or more data packets (e.g., data from one component interacting with another component in a local system, distributed system, and/or across a network, e.g., the Internet, a local area network, a wide area network, etc. with other systems via the signal).

The term "dental feature" or "dental structure" as utilized herein may include intraoral structures or dentition, such as human dentition, individual teeth, quadrants, full arches, upper and lower dental arches (which may be positioned and/or oriented in various occlusal relationships relative to each other), soft tissue (e.g., gingival and mucosal surfaces of the mouth, or perioral structures such as the lips, nose, cheeks, and chin), bones, and any other supporting or surrounding structures proximal to one or more dental structures. Intra-oral structures may include both natural structures within a mouth and artificial structures such as dental objects (e.g., prosthesis, implant, appliance, restoration, restorative component, or abutment). The term "dental feature" may also include a condition or characteristic associated with a dental structure. The condition or characteristic may comprise, for example, (i) a movement of one or more teeth of the subject, (ii) an accumulation of plaque on the one or more teeth of the subject, (iii) a change in a color or a structure of the one or more teeth of the subject, (iv) a change in a color or a structure of a tissue adjacent to the one or more teeth of the subject, (v) a presence or lack of presence of one or more cavities, and/or (vi) an enamel wear pattern. Although the present methods and systems are described with respect to dentition and dental structures, it should be noted that the 3D model construction algorithms and methods described herein can be applied to various other applications where 3D modeling is desired (e.g., 3D modeling of other anatomical or physical features of a human or an animal).

In some cases, artificial intelligence, including machine learning algorithms, may be employed to train a predictive model for image processing, 3D model reconstruction, and various other functionalities as described elsewhere herein. A machine learning algorithm may be a neural network, for example. Examples of neural networks that may be used with embodiments herein may include a deep neural network (DNN), convolutional neural network (CNN), and recurrent neural network (RNN).

In some cases, the predictive model may be trained using supervised learning. In some cases, a machine learning algorithm trained model may be pre-trained and implemented on the physical dental imaging system, and the pre-trained model may undergo continual retraining that may involve continual tuning of the predictive model or a component of the predictive model (e.g., classifier) to adapt to changes in the implementation environment over time (e.g., changes in the image data, model performance, expert input, *etc.).* Alternatively or additionally, the predictive model may be trained using unsupervised learning or semi-supervised learning.

The present disclosure provides methods and systems that are capable of generating (or configured to generate) a high-quality three-dimensional (3D) model of a dental structure of a dental patient using images (e.g., camera image, camera video, *etc*.) collected using a mobile device. The high-quality 3D model may be a 3D surface model (mesh) with fine details of the surface of the dental structure. The high-quality 3D model reconstructed from the camera images may provide a visual representation of the dental structure with a quality, resolution, and/or level of surface details substantially the same or similar as those of 3D models (e.g., optical impressions) produced using a high-resolution clinical dental scanner.

The high-quality 3D model reconstructed from the camera images may preserve the fine surface details obtained from the high-resolution clinical intraoral scan while providing accurate and precise measurements of the current position and orientation of a particular dental structure (e.g., one or more teeth). The clinical high-resolution intraoral scanner can use any suitable intra-oral imaging equipment such as a laser or structured light projection scanner.

### 3D model construction algorithm

In an aspect, the present disclosure provides methods for reconstructing a high quality 3D model of a dental structure. At a first point in time, an initial three-dimensional (3D) model representing a patient's dental structure is provided by a high-quality intraoral scan as described above. In some cases, the initial 3D model includes a 3D surface model with fine surface details. The initial 3D surface model can be obtained using any suitable intraoral scanning device. In some cases, raw point cloud data provided by the scanner may be processed to generate 3D surfaces or point cloud representations of the dental structure (e.g., teeth along with the surrounding gingiva).

At a later point in time during the course of treatment, camera images representing the dental structure may be conveniently captured, obtained, processed, and/or provided using a user mobile device. The camera images are processed to reconstruct a reduced three-dimensional (3D) model of the dental structure. The 3D model may be a 3D point cloud that contains reduced 3D information of the dental structure without fine surface details. In some cases, the 3D model may comprise a dense 3D point cloud. In other cases, the 3D model may comprise a sparse 3D point cloud. A transformation between the reduced three-dimensional (3D) model reconstructed from the camera images and the initial 3D model (mesh model) is determined by aligning or registering elements, feature, or structures of the initial 3D model with corresponding elements, features, or structures within the camera image. A high quality three-dimensional (3D) image of the dental structure is subsequently derived or reconstructed by transforming the initial 3D model using the transformation data. The term "rough 3D model" as utilized herein may generally refer to a 3D model with reduced surface details.

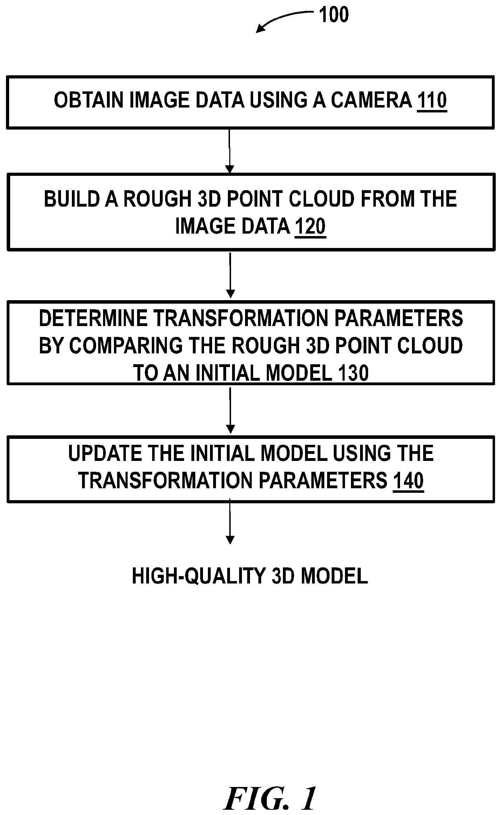

**FIG. 1** shows an example of a 3D model reconstruction algorithm 100, in accordance with some embodiments of the present disclosure. The process may comprise obtaining image data captured using an imaging sensor located at a user device (operation 110). The image data may include a digital representation of at least a portion of the user such as a dental structure or feature of the user. The image data may be intraoral images and/or videos captured using a user device.

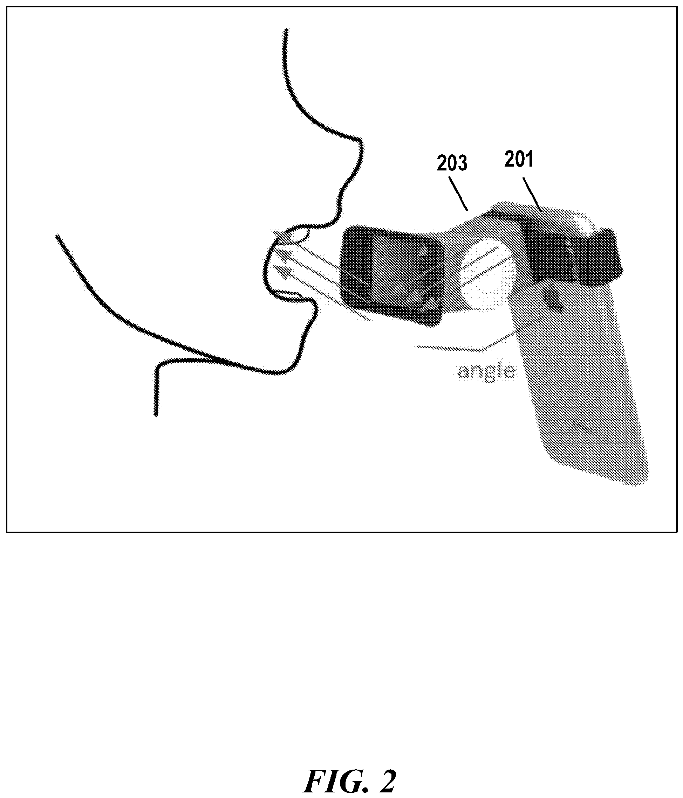

**FIG. 2** shows an example of a user device 201 for capturing the image data. A user may use the mobile device 201 to initiate an intraoral scan. In some cases, the intraoral scan may be performed after an initial clinical intraoral scan has been acquired. The intraoral scan may be performed by a dental patient or a non-professional user at any point in time and at any location. The captured image data may be processed along with the initial 3D surface model to reconstruct a high-quality 3D surface model of the user/subject that accurately reflects the current dental anatomy or dental condition of the subject.

As described above, a dental anatomy may comprise one or more dental structures of the patient, including one or more tooth structures or dental arches of the subject. The dental condition may comprise a development, appearance, and/or condition of the subject's teeth. In some cases, the dental condition may comprise a functional aspect of the user's teeth, such as how two or more teeth contact each other.

In some cases, an intraoral adapter 203 may be used by a user or a subject (e.g., a dental patient) in conjunction with a mobile device to capture the image data. As shown in the example, the intraoral adapter 203 may include a viewing channel of an elongated housing that may be configured to define a field of view of an intraoral region of a subject's mouth. The field of view may be sized and/or shaped to permit one or more cameras of the mobile device to capture one or more images of one or more intraoral regions in a subject's mouth. In some cases, the one or more images may comprise one or more intraoral images showing a portion of a subject's mouth. In some cases, the one or more images may comprise one or more intraoral images showing a full dental arch of the subject.

The mobile device may provide guided instructions for the subject to take one or more intraoral scans. As an example, once a subject reaches a treatment milestone associated with a dental treatment, the intraoral imaging system of the present disclosure may provide the subject with a notification prompting the subject to take an intraoral scan. The subject may connect a mobile device to the intraoral adapter and use the mobile device to initiate an intraoral scan.

For example, a graphical user interface provided on the mobile device 201 may instruct the user to take a plurality of intraoral scans. The plurality of intraoral scans may comprise a left to right or a right to left movement of the intraoral adapter while the user has a closed bite. The plurality of intraoral scans may comprise a left to right or a right to left movement of the intraoral adapter while the user has an open bite. The plurality of intraoral scans may comprise one or more scans of an upper dental arch and/or a lower dental arch of the user. The mobile device (or an application on the mobile device) may assess whether or not the intraoral scans are acceptable, based on lens cleanliness, image clarity, sufficient focus, centering of the intraoral images, and/or whether the subject has achieved a full occlusion capture including internal edges of a left dental arch, a right dental arch, a top dental arch, and/or a bottom dental arch. If an intraoral scan is not acceptable, the subject may be prompted to perform another intraoral scan. If the intraoral scan is acceptable, the mobile device may upload the intraoral scan to a patient's electronic medical record.

### Scan guide

In some embodiments, an artificial intelligence-based scan guide system may be used to help a user or subject capture accurate and comprehensive scans of one or more intraoral features (e.g., dental features, dental structures, and/or dental conditions). Such scans may comprise one or more images or videos of the one or more intraoral features. The artificial intelligence-based scan guide system may be implemented on a mobile device or a mobile computing unit of the user or subject. In some embodiments, the artificial intelligence-based scan guide system may be configured to provide live real-time feedback regarding a position and/or an orientation of one or more cameras of the subject's mobile device relative to one or more intraoral features of the subject (e.g., a dental arch of the subject). In some cases, the live real-time feedback may comprise a visual, audio, or haptic (i.e., vibrational) feedback indicating that the subject's mobile device is in a correct position or orientation for capturing one or more intraoral scans. In other cases, the live real-time feedback may comprise a visual, audio, or haptic (i.e., vibrational) feedback indicating that the subject's mobile device is not in a correct position or orientation for capturing one or more intraoral scans. In some embodiments, the live real-time feedback may comprise a visual, audio, or haptic (i.e., vibrational) feedback indicating a movement, adjustment, or repositioning needed to place the subject's mobile device in a correct position or orientation for capturing one or more intraoral scans.

In some cases, the scan may be divided or discretized into a plurality of stages, and each stage may be used to capture one or more canonical or standardized poses to provide a complete view of the subject's dental arches, including left, right, top, and bottom views of the subject's dental arches. The plurality of stages may comprise at least one, two, three, four, five, six, seven, eight, nine, ten, or more stages. In some cases, each of the plurality of stages may correspond to a distinct canonical or standardized pose. In other cases, each of the plurality of stages may correspond to one or more canonical or standardized poses. In each stage of the plurality of stages, the artificial intelligence-based scan guide system may be configured to search for the relevant canonical view of a subject's teeth in each image or video frame by applying a support-vector machine (SVM) based sliding window detector on an extracted histogram of oriented gradients (HOG) features. The HOG features may comprise feature descriptors that are derived based on a distribution of intensity gradients or edge directions. The HOG features may be derived by dividing the image or video frames of the subject's dental scans into small connected regions or cells and compiling a histogram of gradient directions for the pixels within each cell. The HOG features may correspond to a concatenation of the histograms compiled for one or more pixels of the image or video frames.

In some embodiments, when the artificial intelligence-based scan guide system determines that a relevant canonical view is found, a live feedback may be sent to the user or subject. The live feedback may comprise, for example, a visual stimulation, an auditory stimulation, or a tactile physical stimulation. The visual stimulation may comprise, for example, a flashing of one or more lights of the mobile device, or a flashing of a screen of the mobile device. The auditory stimulation may comprise, for example, an audible tone or sound that is played using one or more speakers of the mobile device. The tactile physical stimulation may comprise, for example, a vibration of the mobile device using one or more vibrational motors of the mobile device.

In some cases, an image processing unit (e.g., cloud application) of the present disclosure may process the intraoral scan to determine a dental condition of the subject. The dental condition may comprise (i) a movement of one or more teeth of the subject, (ii) an accumulation of plaque on the one or more teeth of the subject, (iii) a change in a color or a structure of the one or more teeth of the subject, (iv) a change in a color or a structure of a tissue adjacent to the one or more teeth of the subject, and/or (v) a presence or lack of presence of one or more cavities. In some cases, the image processing unit may use the plurality of intraoral images to (i) predict a movement of one or more teeth of the subject, (ii) identify enamel wear patterns, (iii) create or modify a dental treatment plan, and/or (iv) generate or update an electronic medical record associated with a dental condition of the subject.

The image data can be captured with or without the intraoral adapter. In some cases, the image data may be acquired using any imaging device or user device comprising an imaging sensor. The imaging device may be on-board the user device. The imaging device can include hardware and/or software elements. In some embodiments, the imaging device may be a camera or imaging sensor operably coupled to the user device. In some alternative embodiments, the imaging device may be located external to the user device, and image data of a part of the user may be transmitted to the user device via communication means as described elsewhere herein. The imaging device can be controlled by an application/software configured to take one or more intraoral images or videos of the user. In some embodiments, the camera may be configured to take a 2D image of at least a part of the user's mouth or dental structure. In some embodiments, the software and/or application may be configured to control the camera on the user device to take the one or more intraoral images or videos. In some cases, a plurality of intraoral images from multiple angles may be acquired.

Referring back to **FIG. 1****,** once one or more intraoral images or intraoral videos are obtained, the images or video may be processed to build a reduced 3D model of the dental structure (operation 120). The term "reduced 3D model" may also be referred to as rough model, or sparse model which are used interchangeably throughout the specification.

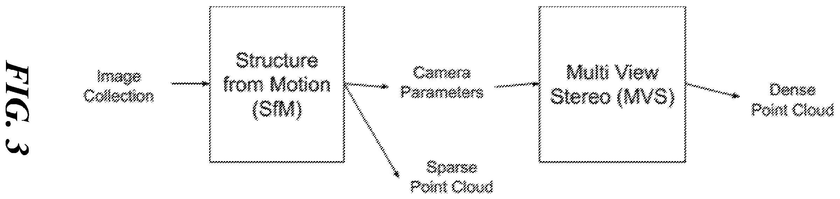

**FIG. 3** shows an exemplary algorithm for building a rough 3D model from the intraoral images or videos. In some cases, the rough 3D model may be a 3D point cloud reconstructed from the image data without fine surface details. As used herein, image data may refer to intraoral images and/or videos obtained using the subject's mobile device.

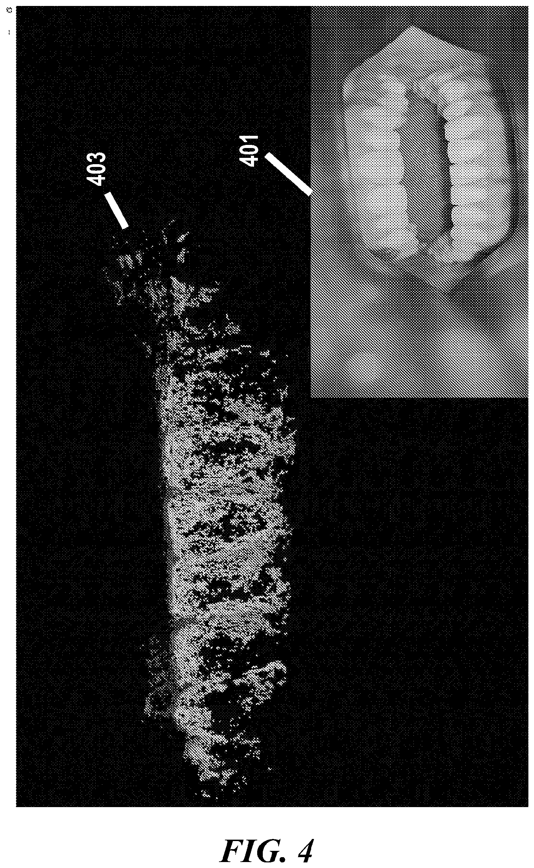

In some cases, the image data collected from the intraoral scan may include images or videos of the dentition (e.g., teeth) from multiple viewing angles. The image data may be processed using any suitable computer vision technique to reconstruct a 3D point cloud of the dental structure. In the illustrated example, the algorithm may include a pipeline for structure from motion (SfM) and multi view stereo (MVS) processing. The first 3D point cloud may be reconstructed by applying structure from motion (SfM) and multi view stereo (MVS) algorithms to the image data. For example, a SfM algorithm is applied to the collected image data to generate estimated camera parameters for each image (and a sparse point cloud describing the scene). Structure from motion (SfM) enables accurate and successful reconstruction in cases where multiple scene elements (e.g., arches) do not move independently of each other throughout the image frames. When these scene elements' movements are substantially independent of each other, segmentation masks may be utilized to track the respective movement. The estimated camera parameters may include both intrinsic parameters such as focal length, focus distance, distance between the micro lens array and image sensor, pixel size, and extrinsic parameters of the camera such as information about the transformations from 3D world coordinates to the 3D camera coordinates. Next, the image data and the camera parameters are processed by the multi-view stereo method to output a dense point cloud of the scene (e.g., a dental structure of a patient). **FIG. 4** shows an example of a rough 3D model (e.g., dense 3D point cloud) 403 reconstructed from the camera image 401. In some cases, the camera images may be segmented such that each point may be annotated with semantic segmentation information.

The rough 3D model (e.g., dense 3D point cloud) can be stored in any suitable file formats such as a Standard Triangle Language (STL) file, a WRL file, a 3MF file, an OBJ, a FBX file, a 3DS file, an IGES file, or a STEP file and various others.

In some cases, pre-processing of the captured image data may be performed to improve the accuracy and quality of the rough 3D model. The pre-processing can include any suitable image processing algorithms, such as image smoothing, to mitigate the effect of sensor noise, image histogram equalization to enhance the pixel intensity values, or image stabilization methods. In some cases, an arch mask may be utilized to track the motion of the arch throughout the video or sequence of images to filter out non-interest anatomical features (e.g., lip, tongue, soft tissue, *etc.)* in the scene. This beneficially ensures that the rough 3D model (e.g., 3D point cloud) substantially corresponds to the surface of the initial 3D model (e.g., teeth and gum).

In some cases, the pre-processing may be performed using machine learning techniques. For example, pixel segmentation can be used to isolate the upper and lower arches and/or mask out the undesired anatomical features. Pixel segmentation may be performed using a deep learning trained model. In another example, image processing such as smoothing, sharpening, stylization may also be performed using a machine learning trained model. The machine learning network can include various types of neural networks including a deep neural network, convolutional neural network (CNN), and recurrent neural network (RNN). The machine learning algorithm may comprise one or more of the following: a support vector machine (SVM), a naive Bayes classification, a linear regression, a quantile regression, a logistic regression, a random forest, a neural network, CNN, RNN, a gradient-boosted classifier or repressor, or another supervised or unsupervised machine learning algorithm (e.g., generative adversarial network (GAN), Cycle-GAN, etc.).

The rough 3D model can be reconstructed using various other methods. For instance, the rough 3D model may be reconstructed from a depth map. In some cases, the imaging device may comprise a camera, a video camera, a three-dimensional (3D) depth camera, a stereo camera, a depth camera, a Red Green Blue Depth (RGB-D) camera, a time-of-flight (TOF) camera, an infrared camera, a charge coupled device (CCD) image sensor, or a complementary metal oxide semiconductor (CMOS) image sensor. The imaging device may be a plenoptic 2D/3D camera, structured light, stereo camera, lidar, or any other camera capable of imaging with depth information.

The imaging device may be used in conjunction with passive or active optical approaches (e.g., structured light, computer vision techniques) to extract depth information about the scene. For example, the depth information or 3D surface reconstruction may be achieved using passive methods that only require images, or active methods that require controlled light to be projected into the surgical site. Passive methods may include, for example, stereoscopy, monocular shape-from-motion, shape-from-shading, optical flow, computational stereo approaches, iterative method combined with predictive models, machine learning approaches, and Simultaneous Localization and Mapping (SLAM) and active methods may include, for example structured light and Time-of-Flight (ToF).

In some cases, the rough 3D model reconstruction method may include generating the three-dimensional model using one or more aspects of passive triangulation. Passive triangulation may involve using stereo-vision methods to generate a three-dimensional model based on a plurality of images obtained using a stereoscopic camera comprising two or more lenses. In other cases, the 3D model construction method may include generating the three-dimensional model using one or more aspects of active triangulation. Active triangulation may involve using a light source (e.g., a laser source) to project a plurality of optical features (e.g., a laser stripe, one or more laser dots, a laser grid, or a laser pattern) onto one or more intraoral regions of a subject's mouth. Active triangulation may involve computing and/or generating a three-dimensional representation of the one or more intraoral regions of the subject's mouth based on a relative position or a relative orientation of each of the projected optical features in relation to one another. Active triangulation may involve computing and/or generating a three-dimensional representation of the one or more intraoral regions of the subject's mouth based on a relative position or a relative orientation of the projected optical features in relation to the light source or a camera of the mobile device.

Machine learning techniques may also be utilized to generate the rough 3D model. For example, one or more operations of the algorithm described in FIG. 3 may be performed using a trained predictive model. For instance, a trained model may be used to generate the camera parameters to replace the structure from motion method.

In another example, a deep learning model may be utilized to process the input raw image data and output a 3D mesh model. For instance, the deep learning model may include a pose estimation algorithm that can reconstruct a 3D surface model using a single image. Alternatively, the 3D surface model may be reconstructed from multiple images. The pose estimation algorithm can be any type of machine learning network such as a neural network.

As an example, the pose estimation algorithm may be an unsupervised learning approach to recover 3D pose from 2D joints/vertices extracted from a single image. The input 2D pose may be the 2D image data captured by the user device camera as described above. The pose estimation algorithm may not require any multi-view image data, correspondences between 2D-3D points, or use of previously learned 3D priors during training. In an example of the pose estimation algorithm, a lifting network may be trained to estimate 3D skeletons from 3D poses. The lifting network may accept 2D landmarks as inputs and generate a corresponding 3D skeleton estimate. During training, the recovered 3D skeleton is re-projected on random camera view-points to generate new 'synthetic' 2D poses. By lifting the synthetic 2D poses back to 3D and re-projecting them in the original camera view, self-consistency loss both in 3D and in 2D may be defined. The training can be selfsupervised by exploiting the geometric self-consistency of the lift-reproject-lift process. The pose estimation algorithm may also comprise a 2D pose discriminator to enable the lifter to output valid 3D poses. In some cases, an unsupervised 2D domain adapter network is trained to allow for an expansion of 2D data. This improves results and demonstrates the usefulness of 2D pose data for unsupervised 3D lifting. The output of the machine learning model may be a 3D mesh model.

The training dataset may include single frame 2D images that are not required from a video. Alternatively, the training dataset may include video data or sequence of images captured from diverse viewpoints. A video may contain one or more objects in one frame performing an array of actions. When video data is available, temporal 2D pose sequences (e.g., video sequence of motions) can improve the accuracy of the signal frame lifting network. Although the pose estimation algorithm described herein uses unsupervised machine learning as an example, it should be noted that the disclosure is not limited thereto, and can use supervised learning and/or other approaches.

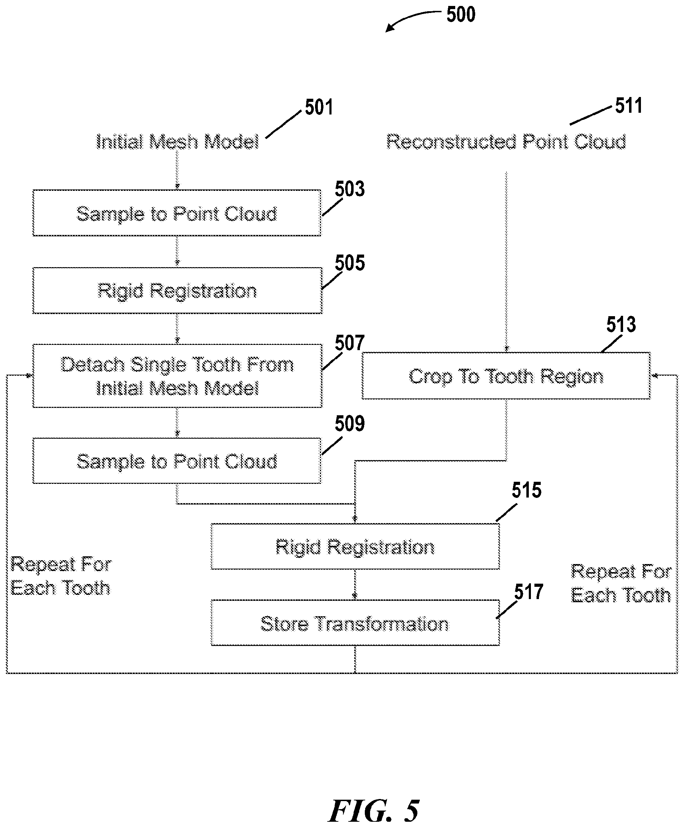

Referring back to **FIG. 1****,** the rough 3D model may be compared to an initial intraoral model of the subject to determine one or more transformation parameters (operation 130). The one or more transformation parameters may define a change of a tooth position relative to the initial position. In some cases, the one or more transformation parameters may define a rigid transformation between a tooth pose in the initial 3D model and a tooth pose in the rough 3D model. The one or more transformation parameters may include translational and rotational deviations or movements. **FIG. 5** shows an example of a method 500 for determining the transformation parameters.

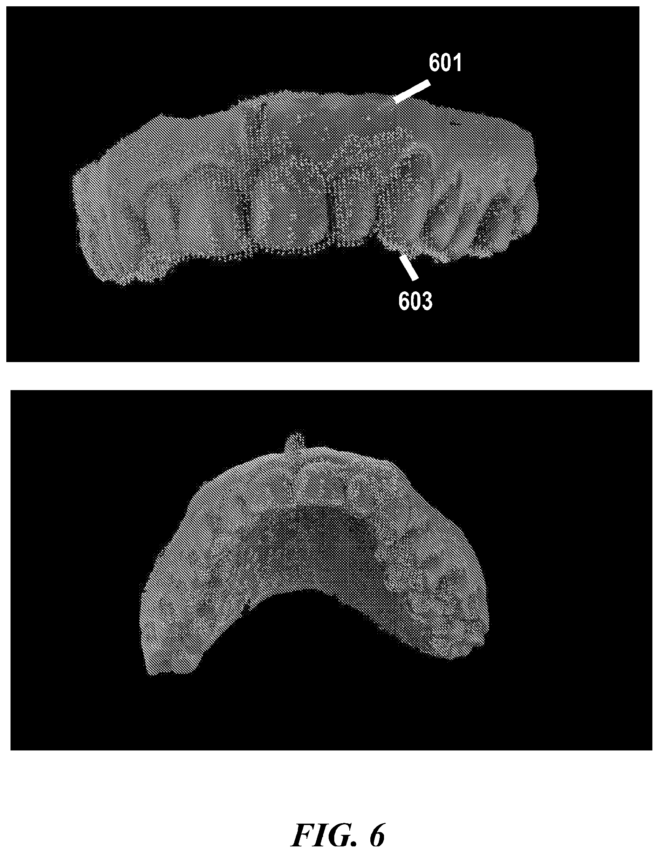

The initial oral model 501 may be a high-quality 3D surface model (mesh) acquired from a high-quality intraoral scanning. For example, the initial oral model 501 can be acquired by a dentist or orthodontist using a dental scanner. The dental scanner may be a 3D intraoral scanner that projects a light source (e.g., laser, structured light) onto the object to be scanned (e.g., dental arches). The images of the dentogingival tissues captured by the imaging sensors may be processed by a scanning software, which generates point clouds. These point clouds are then triangulated by the software to create a 3D surface model (mesh). **FIG. 6** shows an example of a 3D surface model 601 that is obtained from an initial intraoral scan.

Next, a 3D point cloud corresponding to the initial 3D surface model and the reconstructed 3D point cloud from the camera images 511 are processed using a registration algorithm 505. The 3D point cloud corresponding to the initial 3D surface model may be obtained by sampling points from the surface of the 3D model. The sampling may be uniform sampling or non-uniform sampling. Alternatively, the 3D point cloud may be the 3D point cloud directly obtained from the imaging device as described above.

In some cases, the registration algorithm 505 may be used to find a rigid transformation that is applied to the initial 3D point cloud to align it to the rough 3D point cloud 511. As shown in **FIG. 6****,** the rough 3D point cloud 603 is registered with the initial 3D point cloud using a best-fit algorithm such that the rough 3D point cloud is superimposed on the surface of the initial model 601. The registration result may be used to identify one or more elements that have a position change since the initial scan.

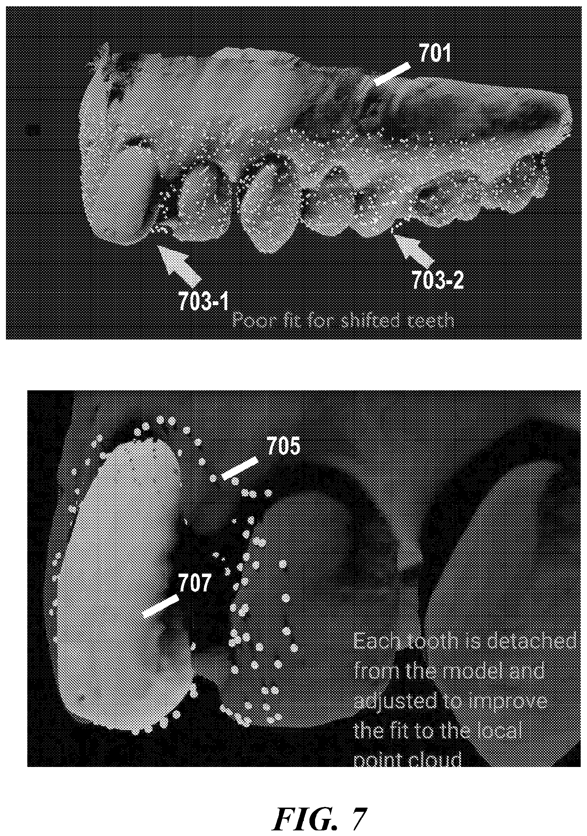

For example, when tooth positions are not changed compared to the initial tooth positions in the initial clinical scan, the rough 3D point cloud 603 may be perfectly superimposed on the surface of the initial model 601 without any mis-matched regions. If a tooth position has changed, an alignment mismatch may be identified (e.g., mismatched region is color-coded in blue, aligned region is color-coded in yellow). **FIG. 7** shows another example of the registration result. After registering the rough 3D point cloud with the initial 3D point cloud, a poor-fit region corresponding to a shifted tooth 701-1, 703-2 is identified. The points on fixed regions (e.g., gums) may serve as anchors which align the rough 3D point cloud reconstruction and the sampled initial 3D point cloud in the same frame of reference.

Once a shifted tooth is identified, a 3D rigid transformation for the identified tooth is determined. The 3D rigid transformation may comprise a translation (change in position with respect to one or more reference axes) and/or a rotation (change in orientation with respect to one or more reference axes). The rigid transformation can be represented as six floating-point numbers.

Referring back to **FIG. 5****,** a rigid transformation for an identified element (e.g., shifted tooth) may be obtained by cropping a region of the element (operation 513) from the reconstructed rough 3D point cloud 511, such that only the points in the vicinity of the element (e.g., tooth) are selected yielding a local target point cloud (e.g., 705 in **FIG. 7**). The corresponding element e.g., tooth (e.g., 707 in **FIG. 7**) is detached from the initial 3D surface model (operation 507) and is sampled to yield an initial local point cloud (operation 509). Next, a rigid transformation (e.g., rotational or translational movement) between the initial local point cloud and the local target point cloud is determined by the rigid registration algorithm (operation 515). The rigid transformation is then stored in a storage device (e.g., operation 517). The process is repeated for every element that has a position change such as the shifted tooth identified as poor-fitting region from the rigid registration result (operation 505).

A tooth may be detached from the initial mesh model based on a mesh segmentation. A segmentation (semantic segmentation) for intra-oral scans (IOS) may comprise labeling all triangles of the mesh as belonging to a specific tooth crown or to gingiva within the recorded IOS point cloud. In some cases, segmentation may comprise assigning labels to various triangles in the mesh. The various triangles may correspond to one or more dental features of the user/subject. A segmentation mask may be used in combination with the segmentation techniques described herein to establish a correspondence between various triangles within two distinct meshes. The various triangles may correspond to a same or similar dental feature. The two distinct meshes may be obtained at different points in time. Any suitable methods can be used for segmenting teeth from the dental model accurately. For example, an end-to-end deep learning framework may be employed for semantic segmentation of individual teeth as well as the gingiva from point clouds representing the initial intra-oral scan. The deep learning approaches may be feature-based deep neural network, volumetric method that voxelizes the shape and applies a 3D CNN model on the quantized shape into a 3D grid space, or a point cloud deep learning model. Alternatively, conventional computer vision algorithms may be utilized for segmentation. For example, the 3D IOS mesh is projected on one or multiple 2D plane(s), then standard computer vision algorithms (e.g., gradient orientation analysis, boundary analysis, curvature analysis, 3D and 3D active contour analysis and tooth-target harmonic fields) are applied, and finally the processed data is projected back into the 3D space. In some embodiments, other registration methods such as deep learning approaches may be employed to determine the rigid transformation.

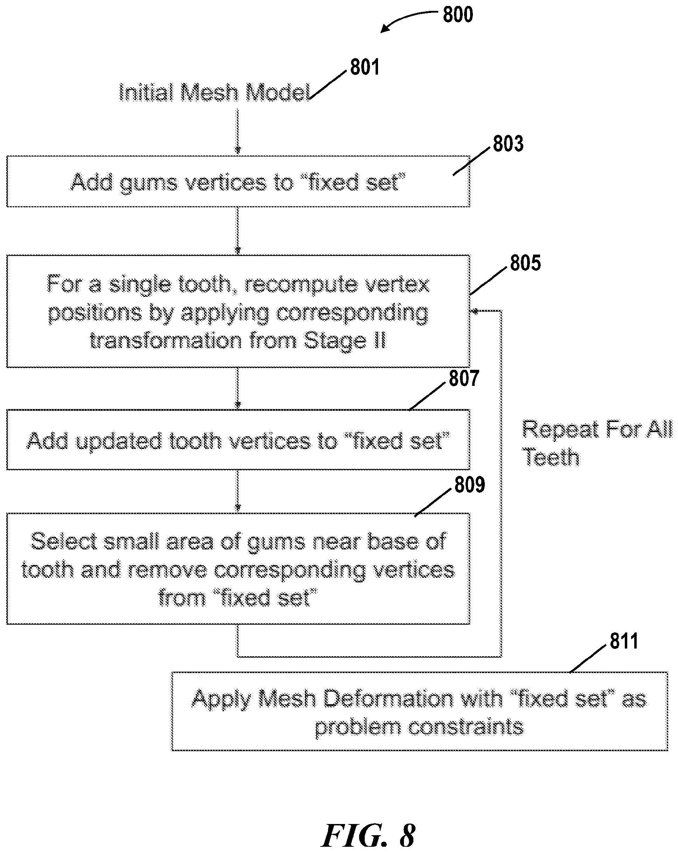

Referring back to **FIG. 1****,** after obtaining the one or more transformation parameters, the initial 3D surface (mesh) model may be updated using a surface deformation algorithm. **FIG. 8** illustrates an example of a surface deformation algorithm 800, in accordance with some embodiments of the present disclosure. The surface deformation algorithm may include an optimization process wherein a set of mesh vertices are constrained to be in fixed regions (e.g., non-shifted teeth and gums) and the position of the "free" vertices are optimized.

As shown in **FIG. 8****,** a set of mesh vertices from the initial mesh model 801 such as vertices from teeth and gums that are fixed in their original position (i.e., said teeth and gums have not changed positions since the initial clinical scanning) are added to a fixed set of surface points (operation 803). The vertices of the shifted tooth are updated to the new positions by applying the rigid transformation obtained from the previous registration process (operation 805). Next, the updated vertices are added to the fixed set (operation 807). Vertices corresponding to a small surface area of the gums surrounding the tooth (e.g., near the base of tooth) are considered as free vertices that the positions can be altered (operation 809). Next, optimization of the free vertices position (mesh deformation) is performed with the fixed set as the optimization problem constraints (operation 811).

A surface deformation algorithm may be applied to deform, for example, the area of the gums surrounding the tooth. In some cases, the area of the gums surrounding a base of a tooth may be bent or stretched to simulate a physical rigid material and preserve the fine surface details. This optimization process can be performed jointly for all teeth or for each tooth sequentially. In some cases, a joint update may be performed for all teeth using a surface deformation algorithm such as an As-Rigid-As-Possible (ARAP) algorithm. Applying the ARAP algorithm may permit shape to be smoothly deformed (e.g., stretched, bent, or sheared) to satisfy the modeling constraints (e.g., fix set of surface points) while allowing small parts of the shape to change as rigidly as possible.

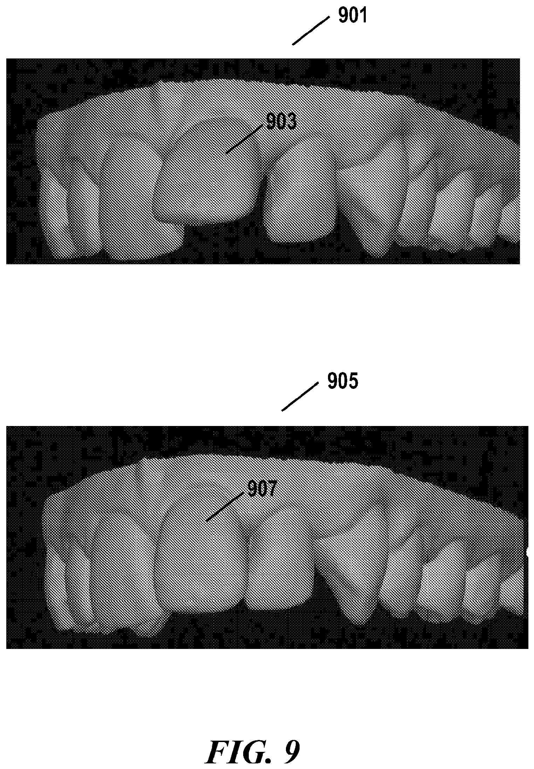

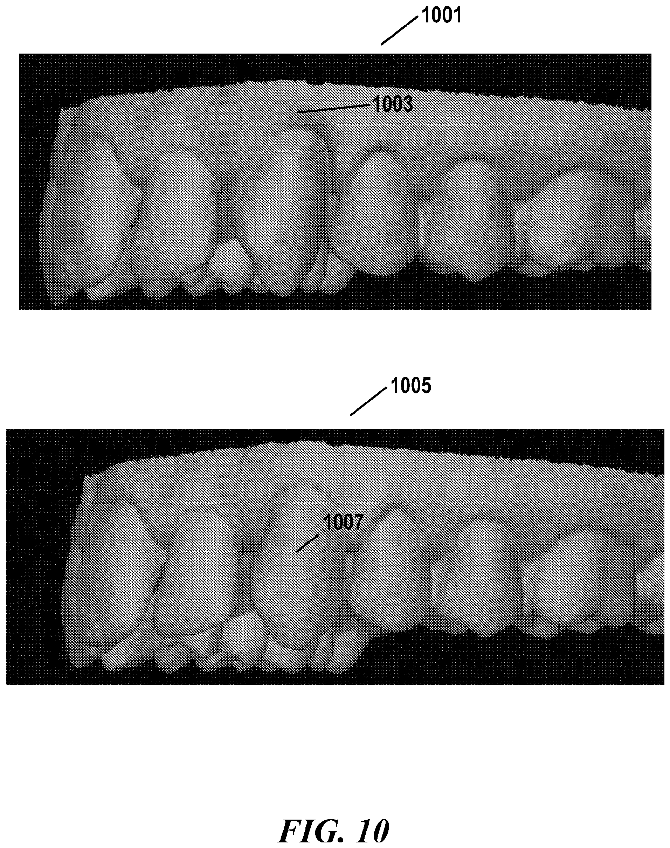

**FIG. 9** shows an example of updating the initial mesh model 901 to generate a new 3D surface model 905 by updating the position of a shifted tooth 903 to the new position 907. **FIG. 10** shows an example of updating the initial mesh model 1001 to generate a new 3D surface model 1005 by optimizing the position and shape of the gum 1003 surrounding a shifted tooth 1007. The gum 1003 may be bent or stretched in the new 3D surface model.

The final output of the method described in **FIG. 1** may be the high-quality 3D surface model (e.g., 905 in **FIG. 9****,** 1005 in **FIG. 10**). Although the method described in **FIG. 1** includes reconstruction of 3D point cloud, other methods that do not require 3D model reconstruction may also be utilized to determine the relative movement of the tooth. For example, the initial 3D mesh model may be rendered as synthetic 2D images and compared with the camera images to determine the rigid transformation in 3D. The position of the tooth in the 3D space may be adjusted interactively until a minimum discrepancy between the pair of 2D images is reached. In some cases, such optimization may be performed using deep learning approaches. In other cases, deep learning may not or need not be used, and the methods of the present disclosure may be implemented using differentiable rendering. Differentiable rendering can be used as a "reconstruction free" alternative to the construction of the first 3D model and subsequent registration of the first 3D model with the initial 3D surface model. Differentiable rendering may be used to perform optimizations using a gradient descent (as opposed to other non-derivative-based optimization methods).

### Reconstruction free methods

In some embodiments, the image processing unit may be configured to implement a "reconstruction-free" method for estimating relative tooth motion. The "reconstruction-free" method results in one or more rigid transformations. The one or more rigid transformation may comprise, for example, a six degree of freedom (DOF) rigid transformation. The relative motion is determined based on a comparison between a 3D scan (e.g., a 3D intraoral scan captured using a clinical dental scanner) and a 2D video scan (e.g., a 2D intraoral video scan captured at a later point in time using a mobile device and any one of the intraoral adapters described herein). The method comprises comparing 2D images from the intraoral scope video to 2D renderings of a 3D mesh, taken from a plurality of different angles. An optimization program is constructed and implemented to adjust the teeth in 3D space such that the 2D renderings match the intraoral video and/or the intraoral images derived from the intraoral video. The level of matching is quantified using an intersection-over-union (IoU) metric. The intersection-over-union (IoU) metric indicates an amount of overlap or similarity between one or more regions within various intraoral images, videos, rendering, and/or 3D models being compared. In some embodiments, differentiable rendering is employed in order to make the optimization amenable to gradient descent, which is used to estimate the tooth motions by solving the optimization program. In some cases, the optimization program may operate based on an assumption that silhouette renderings are sufficient, and binary masks may be extracted from the video frames accordingly. Separately, the camera poses may be derived or estimated from the video frames, in order to support the above procedure. The estimated tooth motions are then used to update the 3D mesh by applying any one or more suitable mesh deformation algorithms as described elsewhere herein.

### Remote dental imaging platform

As used herein, remote monitoring and dental imaging may refer to monitoring a dental anatomy or a dental condition of a patient and taking images of the dental anatomy at one or more locations remote from the patient or dentist. For example, a dentist or a medical specialist may monitor the dental anatomy or dental condition in a first location that is different than a second location where the patient is located. The first location and the second location may be separated by a distance spanning at least 1 meter, 1 kilometer, 10 kilometers, 100 kilometers, 1000 kilometers, or more. The remote monitoring may be performed by assessing a dental anatomy or a dental condition of the subject using one or more intraoral images captured by the subject when the patient is located remotely from the dentist or a dental office. In some cases, the remote monitoring may be performed in real-time such that a dentist is able to assess the dental anatomy or the dental condition when a subject uses a mobile device to acquire one or more intraoral images of one or more intraoral regions in the patient's mouth. The remote monitoring and dental imaging may be performed using equipment, hardware, and/or software that is not physically located at a dental office.

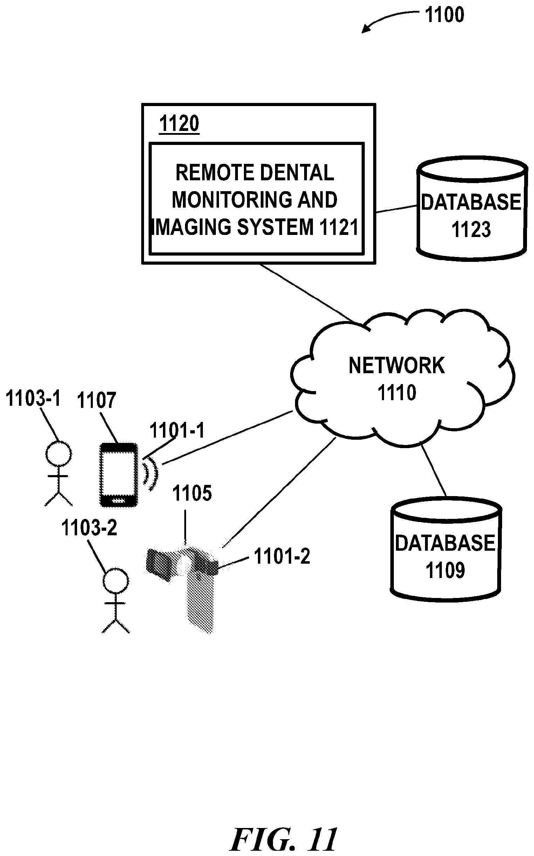

**FIG. 11** illustrates an exemplary environment in which a remote dental monitoring and imaging platform 1100 described herein may be implemented. A remote dental monitoring and imaging platform 1100 may include one or more user devices 1101-1, 1101-2 serving as intraoral imaging systems, a server 1120, a remote dental monitoring and imaging system 1121, and a database 1109, 1123. The remote dental monitoring and imaging platform 1100 may optionally comprise one or more intraoral adapter 1105 that can be used by a user or a subject (e.g., a dental patient) in conjunction with the user device (e.g., mobile device) to remotely monitor a dental anatomy or a dental condition of the subject. Each of the components 1101-1, 1101-2, 1109, 1123, 1120, 1121 may be operatively connected to one another via network 1110 or any type of communication links that allows transmission of data from one component to another.

The remote dental monitoring and imaging system 1121 may be configured to process the input data (e.g., image data) collected from the user device 1101-1, 1101-2 in order to construct a high-quality 3D surface model of the dental anatomy and to provide feedback information (e.g., guidance, diagnosis, treatment plan, quantification result, recommendation) to remotely monitor the dental anatomy or a dental condition of the subject (e.g., development, appearance, and/or condition of the subject's teeth, a functional aspect of the user's teeth, such as how two or more teeth contact each other, etc.). In some cases, the remote dental monitoring and imaging system 1121 may also receive sensor data from the user device to supplement the image data collected by the user device. For example, motion data associated with a movement of the intraoral adapter relative to one or more intraoral regions of interest may be transmitted to the remote dental monitoring and imaging system 1121 along with the image data for 3D model reconstruction. The motion data may be obtained using a motion sensor (e.g., an inertial measurement unit, an accelerometer, a gyroscope, etc.).

The remote dental monitoring and imaging system 1121 may be implemented anywhere within the platform, and/or outside of the platform. In some embodiments, the remote dental monitoring and imaging system may be implemented on the server 1120. In other embodiments, a portion of the remote dental monitoring and imaging system may be implemented on the user device. Alternatively, the remote dental monitoring and imaging system may be implemented in one or more databases. The remote dental monitoring and imaging system may be implemented using software, hardware, or a combination of software and hardware in one or more of the above-mentioned components within the platform.

In some embodiments, one or more components of the platform may reside on the remote entity 1120 (e.g., a cloud). The remote entity 1120 may be a data center, a cloud, a server, and the like that is in communication with one or more user devices, databases, or other third-party entities. In some cases, the remote entity (e.g., cloud) 1120 may include services or applications that run in the cloud or an on-premises environment to remotely monitor the dental condition via the user devices (e.g., 1101-1, 1101-2), imaging sensors 1107, over the network 1110. In some embodiments, the remote entity may host a remote dental monitoring and imaging system 1121 including a plurality of functional components. The plurality of functional components may include at least a 3D model construction module for reconstructing a high-quality 3D surface model, a predictive model management system, cloud applications or other functional components.

For example, the 3D model construction module may be configured to perform the methods, algorithms as described above to reconstruct a high-quality mesh model from camera images. The 3D model construction module may be in communication with the database to retrieve an initial 3D mesh model, and may receive image data from the user device for reconstructing the 3D model using the algorithms and methods as described elsewhere herein.

The cloud applications may include any applications that may utilize the reconstructed 3D model or user applications to guide the user for taking the intraoral scan. For example, the cloud applications may be configured to determine a dental condition of the subject based at least in part on the reconstructed 3D model. The dental condition may comprise: (i) a movement of one or more teeth of the subject, (ii) an accumulation of plaque on the one or more teeth of the subject, (iii) a change in a color or a structure of the one or more teeth of the subject, (iv) a change in a color or a structure of a tissue adjacent to the one or more teeth of the subject, and/or (v) a presence or lack of presence of one or more cavities. In some cases, the reconstructed 3D model may be used to (i) predict a movement of one or more teeth of the subject, (ii) identify enamel wear patterns, (iii) create or modify a dental treatment plan, or (iv) generate or update an electronic medical record associated with a dental condition of the subject. In some cases, the cloud applications may include a dentist application graphical user interfaces (GUI) that allows a caregiver to view the milestone and selfie scans associated with one or more patients and a patient GUI that allows the patient to take an intraoral scan using a user device and upload the images for processing.

As described above, the platform may employ machine learning techniques for image processing. For example, one or more predictive models are trained, developed and deployed for pre-image processing, registration, determining a tooth position change, constructing the 3D surface model, image segmentation, pose estimation, and various others described herein. The remote dental monitoring and imaging system 121 may include a predictive model management system configured to train, develop and manage the various predictive models utilized by the platform.

In some cases, the predictive model management system may comprise a model training module configured to train, develop or test a predictive model using data from the cloud data lake and/or metadata database 1123. The training stage may employ any suitable machine learning techniques that can be supervised learning, unsupervised learning, or semi-supervised learning.

In some cases, model training may use a deep-learning platform to define training applications and to run the training application on a compute cluster. The compute cluster may include one or more GPU-powered servers that may each include a plurality of GPUs, PCIe switches, and/or CPUs, interconnected with high-speed interconnects such as NVLink and PCIe connections. In some examples, a local cache (high-bandwidth scaled out file system) may be available next to the compute cluster and used to cache datasets next to the compute nodes. The system may handle the caching and may provide a local dataset to the compute job. The training applications may produce trained models and metadata that may be stored in a model data store for further consumption. In some cases, the model training process may comprise operations such as model pruning and compression to improve the accuracy and efficacy of the DNNs thereby improving inference speed.

The trained or updated predictive models may be stored in a model database (e.g., database 1123). The model database may contain pre-trained or previously trained models (e.g., DNNs). Models stored in the model database may be monitored and managed by the predictive model management system and continual trained or retrained after deployment. The predictive models created and managed by the remote monitoring and imaging system 1120 may be implemented by the cloud applications and the 3D model construction module.

The remote dental monitoring and imaging system 121 may be hosted on the server 1120. The remote dental monitoring and imaging system may be implemented as a hardware accelerator or as a software executable by a processor.

In some embodiments, one or more systems or components of the present platform are implemented as a containerized application (e.g., application container or service containers). The application container provides tooling for applications and batch processing such as web servers with Python or Ruby, JVMs, or even Hadoop or HPC tooling. The methods and systems can be implemented in application provided by any type of systems (e.g., containerized application, unikernel adapted application, operating-system-level virtualization or machine level virtualization).

The cloud database 1123 may be one or more memory devices configured to store data. Additionally, the databases may also, in some embodiments, be implemented as a computer system with a storage device. In one aspect, the databases may be used by components of the network layout to perform one or more operations consistent with the disclosed embodiments. One or more cloud databases of the platform may utilize any suitable database techniques. For instance, structured query language (SQL) or "NoSQL" database may be utilized for storing the data transmitted from the user device or the local network such as sensor data (e.g., image data, motion data, video data, messages, *etc*.), processed data such as constructed 3D model, dental conditions, predictive model or algorithms. Some of the databases may be implemented using various standard data-structures, such as an array, hash, (linked) list, struct, structured text file (e.g., XML), table, JavaScript Object Notation (JSON), NOSQL and/or the like. Such data-structures may be stored in memory and/or in (structured) files. In another alternative, an object-oriented database may be used. Object databases can include a number of object collections that are grouped and/or linked together by common attributes. The object collections may be related to other object collections by some common attributes. Object-oriented databases perform similarly to relational databases with the exception that objects are not just pieces of data but may have other types of functionality encapsulated within a given object. In some embodiments, the database may include a graph database that uses graph structures for semantic
queries with nodes, edges and properties to represent and store data. If the database of the present invention is implemented as a data-structure, the use of the database of the present invention may be integrated into another component such as the component of the present invention. Also, the database may be implemented as a mix of data structures, objects, and relational structures. Databases may be consolidated and/or distributed in variations through standard data processing techniques. Portions of databases, e.g., tables, may be exported and/or imported and thus decentralized and/or integrated.

The cloud database may comprise storage containing a variety of data consistent with disclosed embodiments. For instance, the databases may store, for example, image data, video data, clinical data (e.g., initial clinical scan, initial mesh model, *etc*.), user profile data (e.g., personal data such as identity, age, gender, contact information, demographic data, ratings, health status, *etc*.), historical data, raw data collected from the user device (e.g., motion data), sensors and wearable device, data about a predictive model (e.g., parameters, hyper-parameters, model architecture, threshold, rules, *etc*.), data generated by a predictive model (e.g., intermediary results, output of a model, latent features, input and output of a component of the model system, *etc*.), and various other data as described elsewhere herein. In some cases, the system 1120 may source data or otherwise communicate (e.g., via the one or more networks 1110) with one or more external systems or data sources 1109, such as healthcare organization platform, Electronic Medical Record (EMR) database, Electronic Health Record (EHR) database and other health authority databases, and the like.

In certain embodiments, one or more of the databases may be co-located with the server 1120, may be co-located with one another on the network, or may be located separately from other devices. One of ordinary skill will recognize that the disclosed embodiments are not limited to the configuration and/or arrangement of the database(s).

The one or more databases (e.g., 1109, 1123) can be accessed by a variety of applications or entities that may utilize the reconstructed 3D model, or require the dental condition. In some cases, the 3D model data stored in the database can be utilized or accessed by other applications through application programming interfaces (APIs). Access to the database may be authorized at per API level, per data level (e.g., type of data), per application level or according to other authorization policies.

Each of the components (e.g., servers, database systems, user devices, external systems, and the like) may be operatively connected to one another via one or more networks 1110 or any type of communication links that allows transmission of data from one component to another. For example, the respective hardware components may comprise network adaptors allowing unidirectional and/or bidirectional communication with one or more networks. For instance, the servers and database systems may be in communicationvia the one or more networks 1110-with the user devices and/or data sources to transmit and/or receive relevant data.

A server may include a web server, a mobile application server, an enterprise server, or any other type of computer server, and can be computer programmed to accept requests (e.g., HTTP, or other protocols that can initiate data transmission) from a computing device (e.g., user device, other servers) and to serve the computing device with requested data. A server may be a unitary server or a distributed server spanning multiple computers or multiple datacenters. The servers may be of various types, such as, for example and without limitation, web server, news server, mail server, message server, advertising server, file server, application server, exchange server, database server, proxy server, another server suitable for performing functions or processes described herein, or any combination thereof. In addition, a server can be a broadcasting facility, such as free-to-air, cable, satellite, and other broadcasting facility, for distributing data. A server may also be a server in a data network (e.g., a cloud computing network).

A server may include various computing components, such as one or more processors, one or more memory devices storing software instructions executed by the processor(s), and data. A server can have one or more processors and at least one memory for storing program instructions. The processor(s) can be a single or multiple microprocessors, field programmable gate arrays (FPGAs), or digital signal processors (DSPs) capable of executing particular sets of instructions. Computer-readable instructions can be stored on a tangible non-transitory computer-readable medium, such as a flexible disk, a hard disk, a CD-ROM (compact disk-read only memory), and MO (magneto-optical), a DVD-ROM (digital versatile disk-read only memory), a DVD RAM (digital versatile diskrandom access memory), or a semiconductor memory. Alternatively, the methods can be implemented in hardware components or combinations of hardware and software such as, for example, ASICs, special purpose computers, or general purpose computers.

The user device 1101-1, 1101-2 may comprise an imaging sensor 1107 serves as imaging device. The imaging device 1107 may be on-board the user device. The imaging device can include hardware and/or software element. In some embodiments, the imaging device may be a camera or imaging sensor operably coupled to the user device. In some alternative embodiments, the imaging device may be located external to the user device, and image data of a dental structure or feature of the user may be transmitted to the user device via communication means as described elsewhere herein. The imaging device can be controlled by an application/software configured to take images or videos of the user's dental structures or features. In some embodiments, the camera may be configured to take a 2D image of at least a portion of the user's dentition. In some embodiments, the software and/or applications may be configured to control the camera on the user device to take one or more intraoral images or videos.

The imaging device 1107 may be a fixed lens or auto focus lens camera. A camera can be a movie or video camera that captures dynamic image data (e.g., video). A camera can be a still camera that captures static images (e.g., photographs). A camera may capture both dynamic image data and static images. A camera may switch between capturing dynamic image data and static images. Although certain embodiments provided herein are described in the context of cameras, it shall be understood that the present disclosure can be applied to any suitable imaging device, and any description herein relating to cameras can also be applied to any suitable imaging device, and any description herein relating to cameras can also be applied to other types of imaging devices. The camera may comprise optical elements (e.g., lens, mirrors, filters, *etc*.). The camera may capture color images (RGB images), greyscale image, and the like.

The imaging device 1107 may be a camera used to capture visual images of at least part of the subject. In some case, the imaging device 1107 may be used in conjunction with an intraoral adapter for performing intraoral scanning. The imaging sensor may collect information anywhere along the electromagnetic spectrum, and may generate corresponding images accordingly.

In some embodiments, the imaging device may be capable of operation at a high resolution. The imaging sensor may have a resolution that is greater than or equal to about 100 µm, 50 µm, 10 µm, 5 µm, 2 µm, 1 µm, 0.5 µm, 0.1 µm, 0.05 µm, 0.01 µm, 0.005 µm, 0.001 µm,0.0005 µm,or 0.0001 µm. The image sensor may be capable of collecting 4K or higher images.

The imaging device 1107 may capture an image frame or a sequence of image frames at a specific image resolution. In some embodiments, the image frame resolution may be defined by the number of pixels in a frame. In some embodiments, the image resolution may be greater than or equal to about 352x420 pixels, 480x320 pixels, 720x480 pixels, 1280x720 pixels, 1440x1080 pixels, 1920x1080 pixels, 2048x1080 pixels, 3840x2160 pixels, 4096x2160 pixels, 7680x4320 pixels, or 15360x8640 pixels.

The imaging device 1107 may capture a sequence of image frames at a specific capture rate. In some embodiments, the sequence of images may be captured at a rate less than or equal to about one image every 0.0001 seconds, 0.0002 seconds, 0.0005 seconds, 0.001 seconds, 0.002 seconds, 0.005 seconds, 0.01 seconds, 0.02 seconds, 0.05 seconds. 0.1 seconds, 0.2 seconds, 0.5 seconds, 1 second, 2 seconds, 5 seconds, or 10 seconds. In some embodiments, the capture rate may change depending on user input and/or external conditions (e.g. illumination brightness).

The imaging device 1107 may be configured to obtain image data to track a motion or a posture of a user. The imaging device may or may not be a 3D camera, stereo camera or depth camera. As described elsewhere herein, computer vision techniques and deep learning techniques may be used to reconstruct 3D pose using the 2D imaging data or generate a depth map. In some cases, the imaging device may be monocular camera and images of the user may be taken from a single view/angle. The imaging device 1107 and the intraoral adapter 1105 can be the same as those described in **FIG. 2****.**

User device 1101-1, 1101-2 may be a computing device configured to perform one or more operations consistent with the disclosed embodiments. Examples of user devices may include, but are not limited to, mobile devices, smartphones/cellphones, tablets, personal digital assistants (PDAs), laptop or notebook computers, desktop computers, media content players, television sets, video gaming station/system, virtual reality systems, augmented reality systems, microphones, or any electronic device capable of analyzing, receiving, providing or displaying certain types of dental related data (e.g., treatment progress, guidance, teeth model, etc.) to a user. The user device may be a handheld object. The user device may be portable. The user device may be carried by a human user. In some cases, the user device may be located remotely from a human user, and the user can control the user device using wireless and/or wired communications.

User device 1101-1, 1101-2 may include one or more processors that are capable of executing non-transitory computer readable media that may provide instructions for one or more operations consistent with the disclosed embodiments. The user device may include one or more memory storage devices comprising non-transitory computer readable media including code, logic, or instructions for performing the one or more operations. The user device may include software applications that allow the user device to communicate with and transfer data between the server 1120, remote dental monitoring and imaging system 1121, and/or database 1109. The user device may include a communication unit, which may permit the communications with one or more other components in the platform 1100. In some instances, the communication unit may include a single communication module, or multiple communication modules. In some instances, the user device may be capable of interacting with one or more components in the platform 1100 using a single communication link or multiple different types of communication links.

User device 1101-1, 1101-2 may include a display. The display may be a screen. The display may or may not be a touchscreen. The display may be a light-emitting diode (LED) screen, OLED screen, liquid crystal display (LCD) screen, plasma screen, or any other type of screen. The display may be configured to show a user interface (UI) or a graphical user interface (GUI) rendered through an application (e.g., via an application programming interface (API) executed on the user device). The GUI may show, for example, a portal for a subject or a dental patient to view one or more intraoral images captured using a mobile device of the subject or the dental patient. In some cases, the user interface may provide a portal for a subject or a dental patient to view one or more three-dimensional models of the subject's or dental patient's dental structure generated based on the one or more intraoral images captured using the mobile device. In some cases, the user interface may provide a portal for a subject or a dental patient to view one or more treatment plans generated based on the one or more intraoral images and/or the one or more three-dimensional models of the subject's dental structure. The portal may be provided through an application programming interface (API). A user or entity can also interact with various elements in the portal via the UI. Examples of UI's include, without limitation, a graphical user interface (GUI) and webbased user interface. The user device may be configured to display webpages and/or websites on the Internet. One or more of the webpages/websites may be hosted by server 1120 and/or rendered by the remote dental monitoring and imaging system 1121.

In some cases, users may utilize the user devices to interact with the remote dental monitoring and imaging system 1121 by way of one or more software applications (i.e., client software) running on and/or accessed by the user devices, wherein the user devices and the remote dental monitoring and imaging system 1121 may form a client-server relationship. For example, the user devices may run dedicated mobile applications or software applications for accessing the patient portal or providing user input.

In some cases, the client software (i.e., software applications installed on the user devices 1101-1, 1101-2) may be available either as downloadable software or mobile applications for various types of computer devices. Alternatively, the client software can be implemented in a combination of one or more programming languages and markup languages for execution by various web browsers. For example, the client software can be executed in web browsers that support JavaScript and HTML rendering, such as Chrome, Mozilla Firefox, Internet Explorer, Safari, and any other compatible web browsers. The various embodiments of client software applications may be compiled for various devices, across multiple platforms, and may be optimized for their respective native platforms.

Server 1120 may be one or more server computers configured to perform one or more operations consistent with the disclosed embodiments. In one aspect, the server may be implemented as a single computer, through which user device are able to communicate with the remote dental monitoring and imaging system and database. In some embodiments, the user device communicates with the remote dental monitoring and imaging system directly through the network. In some embodiments, the server may communicate on behalf of the user device with the remote dental monitoring and imaging system or database through the network. In some embodiments, the server may embody the functionality of one or more of remote dental monitoring and imaging systems. In some embodiments, one or more remote dental monitoring and imaging systems may be implemented inside and/or outside of the server. For example, the remote dental monitoring and imaging systems may be software and/or hardware components included with the server or remote from the server. While **FIG. 11** illustrates the server as a single server, in some embodiments, multiple devices may implement the functionality associated with a server.

Network 1110 may be a network that is configured to provide communication between the various components illustrated in **FIG. 11****.** The network may be implemented, in some embodiments, as one or more networks that connect devices and/or components in the network layout for allowing communication between them. For example, user device 1101-1, 1101-2, and remote dental monitoring and imaging system 1121 may be in operable communication with one another over network 1110. Direct communications may be provided between two or more of the above components. The direct communications may occur without requiring any intermediary device or network. Indirect communications may be provided between two or more of the above components. The indirect communications may occur with aid of one or more intermediary device or network. For instance, indirect communications may utilize a telecommunications network. Indirect communications may be performed with aid of one or more router, communication tower, satellite, or any other intermediary device or network. Examples of types of communications may include, but are not limited to: communications via the Internet, Local Area Networks (LANs), Wide Area Networks (WANs), Bluetooth, Near Field Communication (NFC) technologies, networks based on mobile data protocols such as General Packet Radio Services (GPRS), GSM, Enhanced Data GSM Environment (EDGE), 3G, 4G, 5G or Long Term Evolution (LTE) protocols, Infra-Red (IR) communication technologies, and/or Wi-Fi, and may be wireless, wired, or a combination thereof. In some embodiments, the network may be implemented using cell and/or pager networks, satellite, licensed radio, or a combination of licensed and unlicensed radio. The network may be wireless, wired, or a combination thereof.

User device 1101-1, 1101-2, server 1120, and/or remote dental monitoring and imaging system 1121 may be connected or interconnected to one or more databases 1109, 1123. The databases may be one or more memory devices configured to store data. Additionally, the databases may also, in some embodiments, be implemented as a computer system with a storage device. In one aspect, the databases may be used by components of the network layout to perform one or more operations consistent with the disclosed embodiments. One or more local databases, and cloud databases of the platform may utilize any suitable database techniques as described above.

In some embodiments, the platform may construct the database for fast and efficient data retrieval, query and delivery. For example, the remote dental monitoring and imaging system may provide customized algorithms to extract, transform, and load (ETL) the data. In some embodiments, the remote dental monitoring and imaging system may construct the databases using proprietary database architecture or data structures to provide an efficient database model that is adapted to large scale databases, is easily scalable, is efficient in query and data retrieval, or has reduced memory requirements in comparison to using other data structures.

In one embodiment, the databases may comprise storage containing a variety of data consistent with disclosed embodiments. For example, the databases may store, for example, raw image data collected by the imaging device located on user device. The databases may also store user information, historical data, initial mesh model, medical records, analytics, user input, predictive models, algorithms, training datasets (e.g., video clips), and the like.

In certain embodiments, one or more of the databases may be co-located with the server, may be co-located with one another on the network, or may be located separately from other devices. One of ordinary skill will recognize that the disclosed embodiments are not limited to the configuration and/or arrangement of the database(s).

### Applications

The systems and methods of the present disclosure may be used to perform a variety of applications based on the image/video frames captured and the updated 3D model generated pursuant to the methods described herein.

The systems and methods of the present disclosure may be implemented to perform orthodontic treatment evaluation during treatment. The orthodontic treatment evaluation may comprise a comparison between planned progress and actual progress of the treatment. The orthodontic treatment evaluation may be performed by overlaying a planned stl that was printed for a stage of the treatment with an actual stl that was captured with the intraoral scope during said stage of the treatment. In some instances, the orthodontic treatment evaluation may comprise a digital overlay between two or more stl files, which overlay may allow a dentist to evaluate a patient's compliance with and/or deviation from a prescribed or planned dental treatment plan.

The systems and methods of the present disclosure may be implemented to perform optimization of treatment planning. In such cases, the systems and methods disclosed herein may be configured to use the data set generated from one or more orthodontic treatment evaluations and machine learning capabilities to optimize the way a digital treatment plan is created, adjusted, modified, and/or updated. For conventional systems, a digital treatment planning involves some manual work of a technician and the doctor since a patient usually gets scanned only twice during the treatment and there is insufficient data in the patient's digital/electronic medical record for reliable automated treatment planning. In contrast with such conventional systems, the systems and methods of the present disclosure may be used to create and automatically update digital treatment plans based on a patient's latest treatment progress. Automatically updating a patient's dental treatment plan can ensure that the dental treatment plan (i) more accurately addresses a patient's current treatment needs, and (ii) is tailored to the patient's current dental condition and/or treatment progress to reliably achieve one or more desired treatment goals.

The systems and methods of the present disclosure may be implemented to perform preventive diagnosis for a dental patient or subject. Preventive diagnosis may comprise, for example, detection of plaque, gum recession, color of tooth enamel, enamel wear, and/or cavities. In some cases, the cavities may be visible to the human eye. In other cases, the cavities may not or need not be visible to the human eye.

As described above, in some cases, the 3D surface models described herein may be used to determine a dental condition of a user or patient. The dental condition may comprise (i) a movement of one or more teeth of the subject, (ii) an accumulation of plaque on the one or more teeth of the subject, (iii) a change in a color or a structure of the one or more teeth of the subject, (iv) a change in a color or a structure of a tissue adjacent to the one or more teeth of the subject, and/or (v) a presence or lack of presence of one or more cavities. In some cases, the three-dimensional model may be used to (i) predict a movement of one or more teeth of the subject, (ii) create or modify a dental treatment plan, or (iii) generate or update an electronic medical record based on a current dental condition of the subject or the subject's latest treatment progress. In some cases, the three-dimensional model may be used to track one or more changes in a dental structure or a dental condition of the user or patient over time. In other cases, the three-dimensional model may be used to assess the subject's actual progress in relation to a dental treatment plan based at least in part on a comparison of (i) the one or more changes in the dental structure or the dental condition of the subject and (ii) a planned or estimated change in the dental structure or the dental condition of the subject.

The systems and methods of the present disclosure may be used for remote dental monitoring applications, 3D full-arch simulations based on intraoral scans, treatment overlay comparisons, and smart remote diagnosis (including treatment prediction and automated dental diagnosis). In some cases, the systems and methods of the present disclosure may be used to track the motion of one or more dental features relative to an initial scan, and to update a treatment plan based on the movement of said one or more dental features.

As described above, machine learning algorithms may be employed to train a predictive model for image processing and/or 3D model reconstruction. In some cases, the machine learning algorithms may be configured to use a patient's intraoral scans (and/or any 3D models created based on such intraoral scans) to train a predictive model to (i) generate more accurate predictions of a patient's treatment progress or (ii) generate more accurate predictions of one or more likely treatment outcomes for a patient's dental treatment plan. The machine learning models may be used to predict a course of treatment based on a patient's profile, dental history, treatment progress or treatment outcomes for similar patients, and factors such as a patient's age, gender, ethnicity, genetic profile, dietary profile, and/or existing health conditions. In some cases, the machine learning models may be used to perform feature extraction, feature identification, and/or feature classification for one or more dental features present or visible within a patient's dental scans.

Although particular computing devices are illustrated and networks described, it is to be appreciated and understood that other computing devices and networks can be utilized without departing from scope of the invention, which is defined by the claims. In addition, one or more components of the network layout may be interconnected in a variety of ways, and may in some embodiments be directly connected to, co-located with, or remote from one another, as one of ordinary skill will appreciate.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention, as defined by the claims. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents, within the scope of the claims. It is intended that the following claims define the scope of the invention and that methods and structures within the scope of these claims and their equivalents within the scope of the claims be covered thereby.

## Claims

1. A method (100) for generating a three-dimensional (3D) model of a dental structure of a subject, the method comprising:
(a) capturing image data associated with the dental structure of the subject using a camera of a mobile device (201);
(b) constructing a first 3D model (603, 705) of the dental structure from the image data;
(c) registering the first 3D model (603, 705) with an initial 3D surface model (501, 601) to determine a transformation for at least one element of the dental structure, wherein the initial 3D surface model (501, 601) comprises a mesh with fine surface details, and wherein the first 3D model (603, 705) does not include the fine surface details; and
(d) generating an updated 3D surface model by updating the initial 3D surface model (501, 601), wherein updating the initial 3D surface model (501, 601) comprises at least one of (i) applying the transformation to update a position of the at least one element and (ii) deforming a surface of a local area of the at least one element using a deformation algorithm.

2. The method (100) of claim 1, wherein the first 3D model (603, 705) comprises a first 3D point cloud reconstructed from the image data.

3. The method (100) of claim 2, wherein the image data comprises a sequence of 2D images and the first 3D point cloud is reconstructed by applying a pipeline of structure from motion (SfM) and multi view stereo (MVS) algorithm to the image data.

4. The method (100) of claim 2, wherein the first 3D point cloud is reconstructed by determining one or more camera parameters using a trained model and applying a multi view stereo (MVS) algorithm to the image data using the one or more camera parameters.

5. The method (100) of claim 2, wherein the image data comprises depth data and the first 3D model is reconstructed based on the depth data.

6. The method (100) of claim 1, wherein (c) further comprises generating a second 3D point cloud for the initial 3D surface model and wherein the first 3D model is registered with the second 3D point cloud to identify the at least one element that has a changed position.

7. The method (100) of claim 6, wherein the second 3D point cloud is generated by sampling the surface of the initial 3D surface model.

8. The method (100) of claim 6, wherein the transformation for the at least one element is determined by: (i) selecting a first local point cloud for the at least one element from the first 3D model, (ii) sampling the at least one element from the initial 3D surface model to generate a second local point cloud, and (iii) registering the first local point cloud with the second local point cloud.

9. The method (100) of claim 8, wherein sampling the at least one element from the initial 3D surface model is based on a semantic segmentation of the at least one element.

10. The method (100) of claim 1, wherein the transformation comprises a rotational movement or a translational movement.

11. The method (100) of claim 1, wherein the image data comprises intraoral image data and wherein the method further comprises coupling an intraoral adapter (1105) to the mobile device (201) to facilitate imaging of an intraoral region of the subject's mouth through a viewing channel of the intraoral adapter (1105).

12. The method (100) of claim 1, wherein the image data comprises a plurality of intraoral images, further comprising determining a dental condition of the subject based at least in part on the plurality of intraoral images.

13. The method (100) of claim 2, wherein the image data comprises a sequence of 2D images and the first 3D point cloud is reconstructed using a curve-based reconstruction algorithm.

14. A non-transitory computer-readable medium comprising machine-executable instructions that, upon execution by one or more computer processors, implements a method for delivering context based information to a mobile device in real time, the method comprising:
(a) capturing image data associated with the dental structure of the subject using a camera of a mobile device;
(b) constructing a first 3D model (603, 705) of the dental structure from the image data;
(c) registering the first 3D model (603, 705) with an initial 3D surface model to determine a transformation for at least one element of the dental structure, wherein the initial 3D surface model (501, 601) comprises a mesh with fine surface details, and wherein the first 3D model (603, 705) does not include the fine surface details; and
(d) generating an updated 3D surface model by updating the initial 3D surface model (501, 601), wherein updating the initial 3D surface model (501, 601) comprises at least one of (i) applying the transformation to update a position of the at least one element and (ii) deforming a surface of a local area of the at least one element using a deformation algorithm.

15. A method for generating a three-dimensional (3D) model of a dental structure of a subject, comprising:
(a) capturing image data associated with the dental structure of the subject using a camera of a mobile device (201);
(b) processing the image data using an image processing algorithm, wherein the image processing algorithm is configured to implement differentiable rendering, and wherein processing the image data comprises comparing the image data to one or more two-dimensional (2D) renderings of a three-dimensional (3D) mesh associated with the dental structure of the subject;
(c) using the processed image data to generate a 3D surface model corresponding to one or more dental features represented in the image data;
wherein the step (b) comprises the step (d) of implementing an optimization program to apply one or more rigid transformations to the 3D mesh associated with the dental structure of the subject to align or match at least a portion of the image data to the one or more 2D renderings of the 3D mesh associated with the dental structure of the subject, the rigid transformation representing estimated tooth motion; and
wherein the step (d) comprises the step (e) of evaluating or quantifying a level of matching of the image data to the one or more 2D renderings of the 3D mesh associated with the dental structure of the subject using an intersection-over-union metric to indicate an amount of overlap or similarity between the image data and the one or more 2D renderings of the 3D mesh associated with the dental structure of the subject, and the method further comprises the step (f) of using the estimated tooth motion to update the 3D mesh associated with the dental structure of the subject using one or more mesh deformation algorithms.

## Patentansprüche

1. Verfahren (100) zum Erzeugen eines dreidimensionalen (3D) Modells einer Zahnstruktur eines Subjekts, wobei das Verfahren Folgendes umfasst:
(a) Erfassen von Bilddaten, die sich auf die Zahnstruktur des Subjekts beziehen, unter Verwendung einer Kamera einer Mobilvorrichtung (201);
(b) Erstellen eines ersten 3D-Modells (603, 705) der Zahnstruktur aus den Bilddaten;
(c) Registrieren des ersten 3D-Modells (603, 705) mit einem anfänglichen 3D-Oberflächenmodell (501, 601), um eine Transformation für mindestens ein Element der Zahnstruktur zu bestimmen, wobei das anfängliche 3D-Oberflächenmodell (501, 601) ein Netz mit feinen Oberflächendetails umfasst, und wobei das erste 3D-Modell (603, 705) die feinen Oberflächendetails nicht enthält; und
(d) Erzeugen eines aktualisierten 3D-Oberflächenmodells durch Aktualisieren des anfänglichen 3D-Oberflächenmodells (501, 601), wobei das Aktualisieren des ursprünglichen 3D-Oberflächenmodells (501, 601) mindestens einen der folgenden Schritte umfasst: (i) Anwenden der Transformation, um eine Position des mindestens einen Elements zu aktualisieren, und (ii) Verformen einer Oberfläche eines lokalen Bereichs des mindestens einen Elements unter Verwendung eines Verformungsalgorithmus.

2. Verfahren (100) nach Anspruch 1, wobei das erste 3D-Modell (603, 705) eine erste aus den Bilddaten rekonstruierte 3D-Punktwolke umfasst.

3. Verfahren (100) nach Anspruch 2, wobei die Bilddaten eine Sequenz von 2D-Bildern umfassen und die erste 3D-Punktwolke durch Anwenden einer Pipeline aus Structure-from-Motion-(SfM) und Multi-View-Stereo-(MVS)-Algorithmen auf die Bilddaten rekonstruiert wird.

4. Verfahren (100) nach Anspruch 2, wobei die erste 3D-Punktwolke durch das Bestimmen eines oder mehrerer Kameraparameter unter Verwendung eines trainierten Modells und das Anwenden eines Multi-View-Stereo-Algorithmus (MVS-Algorithmus) auf die Bilddaten unter Verwendung des einen oder der mehreren Kameraparameter rekonstruiert wird.

5. Verfahren (100) nach Anspruch 2, wobei die Bilddaten Tiefendaten umfassen und das erste 3D-Modell basierend auf den Tiefendaten rekonstruiert wird.

6. Verfahren (100) nach Anspruch 1, wobei (c) ferner das Erzeugen einer zweiten 3D-Punktwolke für das anfängliche 3D-Oberflächenmodell umfasst und wobei das erste 3D-Modell mit der zweiten 3D-Punktwolke registriert wird, um das mindestens eine Element zu identifizieren, dessen Position sich geändert hat.

7. Verfahren (100) nach Anspruch 6, wobei die zweite 3D-Punktwolke durch Abtasten der Oberfläche des anfänglichen 3D-Oberflächenmodells erzeugt wird.

8. Verfahren (100) nach Anspruch 6, wobei die Transformation für das mindestens eine Element bestimmt wird durch: (i) Auswählen einer ersten lokalen Punktwolke für das mindestens eine Element aus dem ersten 3D-Modell, (ii) Abtasten des mindestens einen Elements aus dem anfänglichen 3D-Oberflächenmodell, um eine zweite lokale Punktwolke zu erzeugen, und (iii) Registrieren der ersten lokalen Punktwolke mit der zweiten lokalen Punktwolke.

9. Verfahren (100) nach Anspruch 8, wobei das Abtasten des mindestens einen Elements aus dem anfänglichen 3D-Oberflächenmodell auf einer semantischen Segmentierung des mindestens einen Elements basiert.

10. Verfahren (100) nach Anspruch 1, wobei die Transformation eine Rotationsbewegung oder eine Translationsbewegung umfasst.

11. Verfahren (100) nach Anspruch 1, wobei die Bilddaten intraorale Bilddaten umfassen und wobei das Verfahren ferner das Koppeln eines intraoralen Adapters (1105) mit der Mobilvorrichtung (201) umfasst, um die Abbildung eines intraoralen Bereichs des Mundes des Subjekts durch einen Sichtkanal des intraoralen Adapters (1105) zu erleichtern.

12. Verfahren (100) nach Anspruch 1, wobei die Bilddaten eine Vielzahl von intraoralen Bildern umfassen, ferner umfassend das Bestimmen eines Zustands der Zähne des Subjekts basierend zumindest teilweise auf der Vielzahl von intraoralen Bildern.

13. Verfahren (100) nach Anspruch 2, wobei die Bilddaten eine Sequenz von 2D-Bildern umfassen und die erste 3D-Punktwolke unter Verwendung eines kurvenbasierten Rekonstruktionsalgorithmus rekonstruiert wird.

14. Nicht-transitorisches, computerlesbares Medium, das maschinenausführbare Anweisungen umfasst, die bei Ausführung durch einen oder mehrere Computerprozessoren ein Verfahren zur Bereitstellung kontextbasierter Informationen an eine Mobilvorrichtung in Echtzeit implementieren, wobei das Verfahren Folgendes umfasst:
(a) Erfassen von Bilddaten, die sich auf die Zahnstruktur des Subjekts beziehen, unter Verwendung einer Kamera einer Mobilvorrichtung;
(b) Erstellen eines ersten 3D-Modells (603, 705) der Zahnstruktur aus den Bilddaten;
(c) Registrieren des ersten 3D-Modells (603, 705) mit einem anfänglichen 3D-Oberflächenmodell (501, 601), um eine Transformation für mindestens ein Element der Zahnstruktur zu bestimmen, wobei das anfängliche 3D-Oberflächenmodell (501, 601) ein Netz mit feinen Oberflächendetails umfasst, und wobei das erste 3D-Modell (603, 705) die feinen Oberflächendetails nicht enthält; und
(d) Erzeugen eines aktualisierten 3D-Oberflächenmodells durch Aktualisieren des anfänglichen 3D-Oberflächenmodells (501, 601), wobei das Aktualisieren des ursprünglichen 3D-Oberflächenmodells (501, 601) mindestens einen der folgenden Schritte umfasst: (i) Anwenden der Transformation, um eine Position des mindestens einen Elements zu aktualisieren, und (ii) Verformen einer Oberfläche eines lokalen Bereichs des mindestens einen Elements unter Verwendung eines Verformungsalgorithmus.

15. Verfahren zum Erzeugen eines dreidimensionalen (3D) Modells einer Zahnstruktur eines Subjekts, umfassend:
(a) Erfassen von Bilddaten, die sich auf die Zahnstruktur des Subjekts beziehen, unter Verwendung einer Kamera einer Mobilvorrichtung (201);
(b) Verarbeiten der Bilddaten unter Verwendung eines Bildverarbeitungsalgorithmus, wobei der Bildverarbeitungsalgorithmus so konfiguriert ist, dass er ein differenzierbares Rendering implementiert, und wobei das Verarbeiten der Bilddaten das Vergleichen der Bilddaten mit einer oder mehreren zweidimensionalen (2D) Darstellungen eines dreidimensionalen (3D) Netzes umfasst, das sich auf die Zahnstruktur des Subjekts bezieht;
(c) Verwenden der verarbeiteten Bilddaten zur Erzeugung eines 3D-Oberflächenmodells, das einem oder mehreren in den Bilddaten dargestellten Zahnmerkmalen entspricht;
wobei der Schritt (b) den Schritt (d) umfasst, bei dem ein Optimierungsprogramm implementiert wird, um eine oder mehrere starre Transformationen auf das 3D-Netz anzuwenden, das sich auf die Zahnstruktur des Subjekts bezieht, um zumindest einen Teil der Bilddaten an die eine oder die mehreren 2D-Darstellungen des 3D-Netzes, das sich auf die Zahnstruktur des Subjekts bezieht, anzupassen oder damit abzugleichen, wobei die starre Transformation die geschätzte Zahnbewegung darstellt; und
wobei der Schritt (d) den Schritt (e) umfasst, bei dem ein Übereinstimmungsgrad der Bilddaten mit der einen oder den mehreren 2D-Darstellungen des 3D-Netzes, das sich auf die Zahnstruktur des Subjekts bezieht, bewertet oder quantifiziert wird, unter Verwendung einer "Intersection-over-Union"-Metrik, um den Grad der Überlappung oder Ähnlichkeit zwischen den Bilddaten und der einen oder den mehreren 2D-Darstellungen des 3D-Netzes, das sich auf die Zahnstruktur des Subjekts bezieht, anzuzeigen, und wobei das Verfahren ferner den Schritt (f) umfasst, bei dem die geschätzte Zahnbewegung zur Aktualisierung des 3D-Netzes, das sich auf die Zahnstruktur des Subjekts bezieht, verwendet wird, und zwar unter Verwendung eines oder mehrerer Netzverformungsalgorithmen.

## Revendications

1. Procédé (100) de génération d'un modèle tridimensionnel (3D) d'une structure dentaire d'un sujet, le procédé comprenant :
(a) la capture de données d'image associées à la structure dentaire du sujet à l'aide d'une caméra d'un dispositif mobile (201) ;
(b) la construction d'un premier modèle 3D (603, 705) de la structure dentaire à partir des données d'image ;
(c) le calage du premier modèle 3D (603, 705) sur un modèle de surface 3D initial (501, 601) pour déterminer une transformation pour au moins un élément de la structure dentaire, dans lequel le modèle de surface 3D initial (501, 601) comprend un maillage avec des détails de surface fins, et dans lequel le premier modèle 3D (603, 705) ne comporte pas les détails de surface fins ; et
(d) la génération d'un modèle de surface 3D actualisé par actualisation du modèle de surface 3D initial (501, 601), dans lequel l'actualisation du modèle de surface 3D initial (501, 601) comprend (i) l'application de la transformation pour actualiser une position de l'au moins un élément et/ou (ii) la déformation d'une surface d'une zone locale de l'au moins un élément à l'aide d'un algorithme de déformation.

2. Procédé (100) selon la revendication 1, dans lequel le premier modèle 3D (603, 705) comprend un premier nuage de points 3D reconstruit à partir des données d'image.

3. Procédé (100) selon la revendication 2, dans lequel les données d'image comprennent une séquence d'images 2D et le premier nuage de points 3D est reconstruit par application d'un pipeline de structure acquise à partir d'un mouvement (SfM) et d'algorithme de stéréo multi-vue (MVS) aux données d'image.

4. Procédé (100) selon la revendication 2, dans lequel le premier nuage de points 3D est reconstruit par détermination d'un ou de plusieurs paramètres de caméra à l'aide d'un modèle entraîné et application d'un algorithme de stéréo multi-vue (MVS) aux données d'image à l'aide des un ou plusieurs paramètres de caméra.

5. Procédé (100) selon la revendication 2, dans lequel les données d'image comprennent des données de profondeur et le premier modèle 3D est reconstruit sur la base des données de profondeur.

6. Procédé (100) selon la revendication 1, dans lequel le procédé (c) comprend en outre la génération d'un deuxième nuage de points 3D pour le modèle de surface 3D initial et dans lequel le premier modèle 3D est calé sur le deuxième nuage de points 3D pour identifier l'au moins un élément qui a une position changée.

7. Procédé (100) selon la revendication 6, dans lequel le deuxième nuage de points 3D est généré par échantillonnage de la surface du modèle de surface 3D initial.

8. Procédé (100) selon la revendication 6, dans lequel la transformation pour l'au moins un élément est déterminée par : (i) sélection d'un premier nuage de points local pour l'au moins un élément à partir du premier modèle 3D, (ii) échantillonnage de l'au moins un élément à partir du modèle de surface 3D initial pour générer un deuxième nuage de points local, et (iii) calage du premier nuage de points local sur le deuxième nuage de points local.

9. Procédé (100) selon la revendication 8, dans lequel l'échantillonnage de l'au moins un élément à partir du modèle de surface 3D initial est basé sur une segmentation sémantique de l'au moins un élément.

10. Procédé (100) selon la revendication 1, dans lequel la transformation comprend un déplacement en rotation ou un déplacement en translation.

11. Procédé (100) selon la revendication 1, dans lequel les données d'image comprennent des données d'image intrabuccale et dans lequel le procédé comprend en outre l'accouplement d'un adaptateur intrabuccal (1105) au dispositif mobile (201) pour faciliter l'imagerie d'une région intrabuccale de la bouche du sujet à travers un canal de visualisation de l'adaptateur intrabuccal (1105).

12. Procédé (100) selon la revendication 1, dans lequel les données d'image comprennent une pluralité d'images intrabuccales, comprenant en outre la détermination d'une affection dentaire du sujet sur la base au moins en partie de la pluralité d'images intrabuccales.

13. Procédé (100) selon la revendication 2, dans lequel les données d'image comprennent une séquence d'images 2D et le premier nuage de points 3D est reconstruit à l'aide d'un algorithme de reconstruction basé sur des courbes.

14. Support non transitoire lisible par ordinateur comprenant des instructions exécutables par machine qui, lors d'une exécution par un ou plusieurs processeurs d'ordinateur, mettent œuvre un procédé destiné à délivrer des informations basées sur un contexte à un dispositif mobile en temps réel, le procédé comprenant :
(a) la capture de données d'image associées à la structure dentaire du sujet à l'aide d'une caméra d'un dispositif mobile ;
(b) la construction d'un premier modèle 3D (603, 705) de la structure dentaire à partir des données d'image ;
(c) le calage du premier modèle 3D (603, 705) sur un modèle de surface 3D initial pour déterminer une transformation pour au moins un élément de la structure dentaire, dans lequel le modèle de surface 3D initial (501, 601) comprend un maillage avec des détails de surface fins, et dans lequel le premier modèle 3D (603, 705) ne comporte pas les détails de surface fins ; et
(d) la génération d'un modèle de surface 3D actualisé par actualisation du modèle de surface 3D initial (501, 601), dans lequel l'actualisation du modèle de surface 3D initial (501, 601) comprend (i) l'application de la transformation pour actualiser une position de l'au moins un élément et/ou (ii) la déformation d'une surface d'une zone locale de l'au moins un élément à l'aide d'un algorithme de déformation.

15. Procédé de génération d'un modèle tridimensionnel (3D) d'une structure dentaire d'un sujet, comprenant :
(a) la capture de données d'image associées à la structure dentaire du sujet à l'aide d'une caméra d'un dispositif mobile (201) ;
(b) le traitement des données d'image à l'aide d'un algorithme de traitement d'image, dans lequel l'algorithme de traitement d'image est configuré pour mettre en œuvre un rendu différentiable, et dans lequel le traitement des données d'image comprend la comparaison des données d'image à un ou plusieurs rendus bidimensionnels (2D) d'un maillage tridimensionnel (3D) associé à la structure dentaire du sujet ;
(c) l'utilisation des données d'image traitées pour générer un modèle de surface 3D correspondant à une plusieurs caractéristiques dentaires représentées dans les données d'image ;
dans lequel l'étape (b) comprend l'étape (d) consistant à mettre en œuvre un programme d'optimisation pour appliquer une ou plusieurs transformations rigides au maillage 3D associé à la structure dentaire du sujet afin d'aligner ou de faire concorder au moins une portion des données d'image avec les un ou plusieurs rendus 2D du maillage 3D associé à la structure dentaire du sujet, la transformation rigide représentant un mouvement de dent estimé ; et
dans lequel l'étape (d) comprend l'étape (e) consistant à évaluer ou quantifier un niveau de concordance des données d'image avec les un ou plusieurs rendus 2D du maillage 3D associé à la structure dentaire du sujet à l'aide d'une métrique d'intersection sur union afin d'indiquer une quantité de chevauchement ou de similitude entre les données d'image et les un ou plusieurs rendus 2D du maillage 3D associé à la structure dentaire du sujet, et le procédé comprend en outre l'étape (f) consistant à utiliser le mouvement de dent estimé pour actualiser le maillage 3D associé à la structure dentaire du sujet à l'aide d'un ou de plusieurs algorithmes de déformation de maillage.
